# EUROPÄISCHE PATENTSCHRIFT

(11) **EP 2 359 765 B1**
(45) Veröffentlichungstag und Bekanntmachung des Hinweises auf die Patenterteilung: **13.09.2017**
(21) Anmeldenummer: 11153701.5
(22) Anmeldetag: 08.02.2011
(51) Int. Cl.: A61B 18/14, A61B 18/16

(54) **HF-Chirurgiegerät**
High frequency surgical device
Appareil de chirurgie HF

(30) Priorität: 12.02.2010 DE 102010000396
(43) Veröffentlichungstag der Anmeldung: 24.08.2011
(73) Patentinhaber: Erbe Elektromedizin GmbH, 72072 Tübingen (DE)
(72) Erfinder: Hagg, Martin, 72827 Wannweil (DE); Selig, Peter, 72379 Hechingen (DE)
(74) Vertreter: Bohnenberger, Johannes

(56) Entgegenhaltungen:
- WO-A1-02/19932
- WO-A1-2004/062516
- US-A- 4 121 590

## Beschreibung

Die Erfindung betrifft ein HF-Chirurgiegerät gemäß dem Oberbegriff des Anspruchs 1.

HF-Chirurgiegeräte der hier angesprochenen Art sind bekannt. Sie weisen einen HF-Generator und wenigstens ein chirurgisches Instrument mit einer aktiven Elektrode auf, die mit dem HF-Generator über eine Leitung zur Zuführung eines hochfrequenten Stroms verbunden ist. Weiterhin ist eine Neutralelektrode vorgesehen, die ebenfalls mit dem HF-Generator über eine entsprechende zweite Leitung zur Rückführung des Stroms verbunden ist.

Ein HF-Generator der hier angesprochenen Art erzeugt eine hochfrequente elektrische Spannung bzw. Ströme und leitet diese über eine entsprechende Leitung zu dem angeschlossenen chirurgischen Instrument. Bei dem chirurgischen Instrument kann es sich um ein monopolares, bipolares oder quasi bipolares Instrument zum Schneiden und/oder Koagulieren von biologischem Gewebe handeln. Weiterhin können Zubehöreinrichtungen zur Einstellung, Überwachung, Regelung, Begrenzung und/oder Modulation der zum Schneiden und/oder Koagulieren erforderlichen HF-Spannungen, HF-Ströme, elektrische Lichtbögen zwischen aktiver Elektrode und biologischem Gewebe mit dem HF-Generator verbunden oder in diesem angeordnet sein.

Mit derartigen HF-Chirurgiegeräten sind jedoch Anforderungen verbunden, die sich z.B. in der Richtlinie 2004/40/EG vom 29. April 2004 des Europäischen Parlaments über Mindestvorschriften zum Schutz von Sicherheit und Gesundheit der Arbeitnehmer vor der Gefährdung durch physikalische Einwirkungen (elektromagnetische Felder) niederschlagen. Diese erfordern bei HF-Chirurgiegeräten eine abgeschirmte Ausbildung von Leitungen, die zu einem Patienten führen, um unter anderem die durch den hochfrequenten Strom entstehenden elektrischen Felder zu minimieren. Eine gattungsgemäße HF-Chirurgieeinrichtung mit abgeschirmten Leitungen ist beispielsweise aus der DE 91 17 217 U1 bekannt. Nachteilig ist es hierbei, dass die Verwendung von abgeschirmten Leitungen zur Verbindung des chirurgischen Instruments mit dem HF-Generator bzw. zur Verbindung der Neutralelektrode mit dem HF-Generator mit nicht unerheblichen Problemen verbunden ist.

Durch die Verwendung von geschirmten Leitungen ergibt sich insbesondere das Problem, dass die Abschirmung eine kapazitive Last bildet, welche die Höhe der Ausgangsspannung zwischen dem chirurgischen Instrument und dem zu behandelnden Gewebe vermindert. Für den gewünschten HF-chirurgischen Effekt ist die Höhe der zwischen der aktiven Elektrode und dem zu behandelnden Gewebe anliegenden hochfrequenten Spannung jedoch ein maßgeblicher Parameter. Weiterhin ist es notwendig bzw. durch die einschlägigen Normen vorgegeben, dass im Ausgangskreis des HF-Generators ein so genannter Auskoppelkondensator vorgesehen ist, um die Entstehung von Gleichstromkomponenten zu unterdrücken, die schädlich für den Patienten sein können.

Weiterhin ergibt sich durch die Verwendung von abgeschirmten Leitungen das Problem, dass die Abschirmung der Leitungen vorzugsweise geerdet ist und durch diesen Erdbezug der Abschirmung die Entstehung von Leckströmen an Stellen begünstigt wird, an denen der Patient mit geerdeten, leitfähigen Teilen in Kontakt steht, insbesondere können diese Leckströme größer werden, was sich wiederum ungünstig auf den chirurgischen Effekt auswirkt und darüber hinaus eine Gefahr für den Patienten darstellt. US4121590 offenbart ein HF-Chirurgiegerät mit HF-Generator, chirurgisches Instrument mit aktiver Elektrode, und eine Neutralelektrode die mit dem HF-Generator verbunden sind, wobei das chirurgische Instrument mit dem HF-Generator über eine erste geerdete abgeschirmte Leitung und die Neutralelektrode mit dem HF-Generator über eine zweite Leitung verbunden ist. Aufgabe der vorliegenden Erfindung ist es daher, ein HF-Chirurgiegerät zu schaffen, welches die Probleme, die bei der Verwendung von abgeschirmten Leitungen auftreten vermeidet oder zumindest vermindert, das also einerseits eine Verminderung der Ausgangsspannung am Einsatzort des chirurgischen Instruments vermeidet und welches andererseits die Entstehung von Leckströmen vermindert.

Zur Lösung dieser Aufgabe wird ein HF-Chirurgiegerät mit den Merkmalen des Anspruchs 1 vorgeschlagen. Es umfasst einen HF-Generator und wenigstens ein chirurgisches Instrument mit einer aktiven Elektrode sowie eine Neutralelektrode. Das chirurgische Instrument ist über eine erste Leitung mit dem HF-Generator und die Neutralelektrode mit einer zweiten Leitung mit dem HF-Generator verbindbar. Die erste und/oder die zweite Leitung zur Verbindung des chirurgischen Instruments und der Neutralelektrode mit dem HF-Generator sind mit einer Abschirmung versehen, wobei an dem distalen Ende der jeweils abgeschirmten Leitung ein Auskoppelkondensator angeordnet ist. Die Leitung(en), die mit einer Abschirmung versehen ist (sind), weisen also an ihrem distalen Ende einen Auskoppelkondensator auf. Gemäß der Erfindung wird der Auskoppelkondensator des HF-Generators somit in vorteilhafter Weise nicht in dem Generatorgehäuse untergebracht, sondern vielmehr erst an dem distalen Ende der Leitungen zur Verbindung der aktiven Elektrode und der Neutralelektrode mit dem HF-Generator. Auf diese Weise werden die eingangs beschriebenen Nachteile der verwendeten Leitungsabschirmung ausgeräumt oder zumindest wesentlich verringert.

Besonders bevorzugt ist das HF-Chirurgiegerät mit Leitungen versehen, deren Abschirmung über einen Erdungspfad geerdet ist. Ein über den Erdungspfad fließender Leckstrom kann dann besonders bevorzugt mittels einer Strommessvorrichtung erfasst werden.

Vorzugsweise ist sowohl die erste und die zweite Leitung mit einer Abschirmung versehen, so dass dann an dem distalen Ende der ersten Leitung und an dem distalen Ende der zweiten Leitung jeweils ein Auskoppelkondensator vorgesehen ist. Es kann aber auch vorgesehen sein, dass ausschließlich an dem distalen Ende der zweiten Leitung ein Auskoppelkondensator vorgesehen ist oder ausschließlich an dem distalen Ende der ersten Leitung ein Auskoppelkondensator vorgesehen ist.

Schließlich wird auch ein HF-Chirurgiegerät bevorzugt, bei dem die Abschirmungen der Leitungen an einem zentralen Sternpunkt über Rückleitungen mit dem HF-Generator verbindbar sind. Zur Vermeidung bzw. Verminderung von Leckströmen bzw. ihre Störfelder ist in den Rückleitungen der Abschirmungen vorzugsweise wenigstens eine Gleichtaktdrossel vorgesehen. Durch die Rückleitungen ergibt sich der Vorteil, dass den Leckströmen ein niederimpedanter Weg innerhalb des Gerätes angeboten werden kann, sodass Strompfade an Stellen, an denen der Patient mit geerdeten, leitfähigen Teilen in Kontakt ist, vermieden bzw. vermindert werden.

Die Erfindung wird im Folgenden anhand der Zeichnung näher erläutert. Es zeigen:
- Fig. 1: ein schematisches Schaltbild eines HF-Chirurgiegeräts gemäß der Erfindung;
- Fig. 2: ein Ersatzschaltbild des HF-Chirurgiegeräts nach Fig. 1;
- Fig. 3: ein schematisches Schaltbild eines HF-Chirurgiegeräts nach einer Ausführungsform der Erfindung, und
- Fig. 4: ein schematisches Schaltbild eines HF-Chirurgiegeräts nach einer weiteren Ausführungsform der Erfindung.

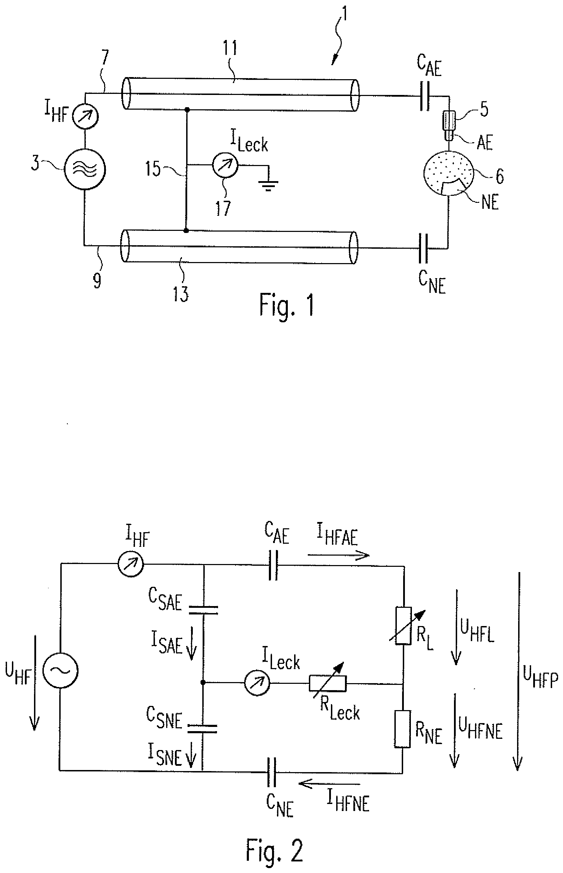

Fig. 1 zeigt ein schematisches Schaltbild eines HF-Chirurgiegeräts 1 gemäß der Erfindung. Das HF-Chirurgiegerät 1 weist einen HF-Generator 3 sowie ein hier nur angedeutet dargestelltes chirurgisches Instrument 5 auf, bei dem es sich beispielsweise um ein chirurgisches Instrument zum Plasmakoagulieren von biologischem Gewebe handeln kann. Das chirurgische Instrument 5 weist eine aktive Elektrode AE auf, die in der Nähe eines zu behandelnden Gewebes 6 angeordnet ist. An dem zu behandelnden Gewebe 6 ist weiterhin eine Neutralelektrode NE vorgesehen, die zur Rückführung eines Stromflusses durch den Patienten in den HF-Generator 3 dient und hierzu mit dem Patienten in Kontakt steht.

Die aktive Elektrode AE des chirurgischen Instruments 5 ist über eine erste Leitung 7 mit dem HF-Generator 3 zur Zuführung eines hochfrequenten Stroms verbunden. Die Neutralelektrode NE ist mittels einer zweiten Leitung 9 ebenfalls mit dem HF-Generator 3 zur Rückführung des Stroms verbunden. In der Ausführungsform gemäß Fig. 1 weist die erste Leitung 7 eine Abschirmung 11 und die zweite Leitung 9 eine Abschirmung 13 auf, um die Abstrahlung von elektrischen Feldern durch die Leitung(en) 7 und/oder 9 zu minimieren.

Die Abschirmungen 11 und 13 sind vorzugsweise, wie in der Fig. 1 dargestellt, über einen Erdungspfad 15 geerdet, wobei die Erdung gemäß Fig. 1 rein beispielhaft über den gemeinsamen Erdungspfad 15 erfolgt. Es versteht sich, dass auch getrennte Erdungspfade der beiden Abschirmungen vorgesehen sein können. Weiterhin ist im Erdungspfad 15 eine Strommessvorrichtung 17 vorgesehen, welche den Leckstrom I_{Leck} misst. Die Leckströme lassen sich mit dieser Anordnung relativ leicht messen, da ihnen nur der Weg über die Erdung des HF-Chirurgiegeräts 1 inklusive der Erdung der Abschirmungen 11 und 13 bleibt und die Leckströme somit praktisch vollständig gemessen werden können. Bei ungeschirmten Leitungen besteht dagegen das Problem, dass die Leckströme auf kapazitivem Weg in die außerhalb des Gerätes liegenden Patientenleitungen fließen und somit von einer Sensorik im Gerät nicht erfasst werden können. Selbst wenn also die Auftrittswahrscheinlichkeit von Leckströmen nicht verringert werden kann, lassen sich die Leckströme jedoch durch die hier vorgeschlagene wenigstens eine erfindungsgemäße Strommessvorrichtung 17 in den Erdungspfaden der Abschirmungen zumindest erfassen.

Am distalen Ende der Leitung 7 ist gemäß der Erfindung ein Auskoppelkondensator C_{AE} vorgesehen, der das einleitend erstgenannte Problem eines ungünstigen kapazitiven Spannungsteilers durch die kapazitive Last der Abschirmung 11 vermeidet. Der ungünstige kapazitive Spannungsteiler reduziert insbesondere im hochohmigen Lastfall oder im Leerlauf die zwischen der aktiven Elektrode AE und der Neutralelektrode NE liegende HF-Spannung U_{HFP} gegenüber der Generatorspannung U_{HF}. Durch die Verlagerung des Auskoppelkondensators C_{AE} an das distale Ende der ersten Leitung 7 wird dieses Problem deutlich reduziert, insbesondere bei Anwendungen, bei denen hohe Leerlaufspannungen benötigt werden, wie beispielsweise bei der Spraykoagulation, bei der Argon-Plasma-Koagulation o.ä..

In der Ausführungsform gemäß Fig. 1 weist auch das distale Ende der zweiten Leitung 9, die mit der Neutralelektrode NE verbunden ist, einen Auskoppelkondensator C_{NE} auf, wodurch eine weitere Verbesserung der mit der Abschirmung einhergehenden Probleme erzielt werden kann. Durch die relativ großflächige Neutralelektrode NE ist es besonders einfach, dort einen Auskoppelkondensator vorzusehen.

Insgesamt wird deutlich, dass die Probleme, welche mit den Abschirmungen 11 und 13 der Leitungen 7 und 9 einhergehen, durch die Verlagerung des Auskoppelkondensators des HF-Generators 3 aus dem Generatorgehäuse heraus an ein distales Ende der Leitungen 7 und 9 vermieden werden können.

Fig. 2 zeigt ein Ersatzschaltbild des HF-Chirurgiegeräts 1 gemäß der Fig. 1, welches den oben beschriebenen Spannungsteiler zeigt. Die kapazitive Last der Abschirmungen 11 und 13 sind durch die seriellen Kapazitäten C_{SAE} und C_{SNE} dargestellt. Der Lastwiderstand R_{L} ist in der Fig. 2 als regelbarer Widerstand ausgeführt, der die verschiedenen Lastzustände, insbesondere einen hochohmigen Lastfall oder einen Leerlauf verdeutlichen soll. Weiterhin ist die Impedanz der Neutralelektrode NE durch den Widerstand R_{NE} dargestellt. Der regelbare Widerstand R_{Leck} macht deutlich, dass der Leckstrom insbesondere in Abhängigkeit des Widerstands R_{L} veränderbar ist.

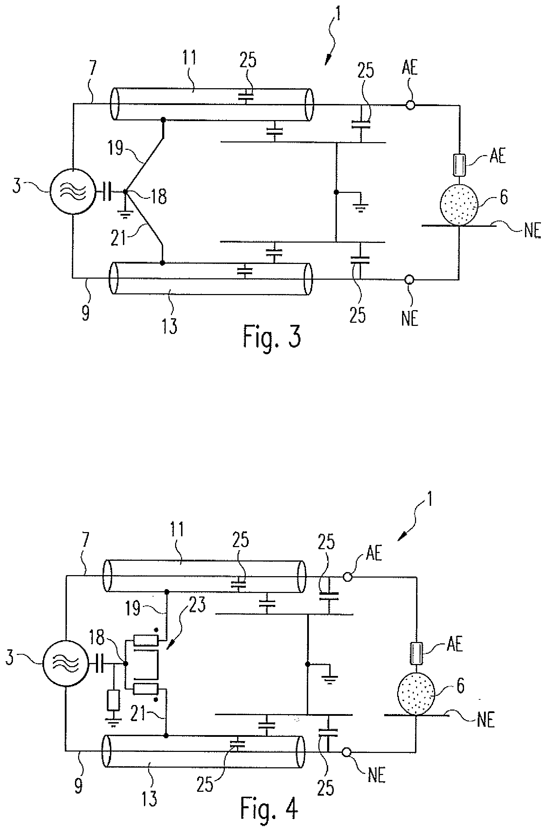

Fig. 3 zeigt eine weitere Ausführungsform des HF-Chirurgiegeräts 1, bei dem die Abschirmungen 11 und 13 über Rückleitungen 19 und 21 mit dem HF-Generator 13 verbunden sind. Dabei ist jeweils eine Abschirmung 11, 13 mit einer Rückleitung 19, 21 verbunden, die in einem gemeinsamen zentralen Sternpunkt 18 zusammenlaufen, der mit dem HF-Generator über eine Kapazität verbunden ist. Dadurch wird den Leckströmen ein niederimpedanter Weg innerhalb des HF-Chirurgiegerätes angeboten, wodurch das Auftreten von Leckströmen an unerwünschten Stellen weitgehend vermieden werden kann.

Besonders bevorzugt wird, wie in der Fig. 4 dargestellt ist, wenigstens eine Gleichtaktdrossel 23 in den Rückleitungen 19 und 21 angeordnet. Die Gleichtaktdrossel 23 hat vorzugsweise mehrere gleiche Wicklungen, die gegensinnig vom Leckstrom in den Rückleitungen 19 und 21 durchflossen werden, sodass sich deren magnetische Felder im Kern der Drossel aufheben. Hierdurch wird eine Dämpfung von Störemissionen der Leckströme erreicht.

In den Fig. 3 und 4 sind darüber hinaus noch kapazitive Kopplungen dargestellt, insbesondere (parasitäre) Kapazitäten 25, die zwischen den Leitungen und den Abschirmungen bzw. zwischen den Abschirmen und anderen in der Umgebung befindlichen Elementen entstehen.

### Bezugszeichenliste

- 1: HF-Chirurgiegerät
- 3: HF-Generator
- 5: Chirurgisches Instrument
- 6: Gewebe
- 7: erste Leitung
- 9: zweite Leitung
- 11: Abschirmung
- 13: Abschirmung
- 15: Erdungspfad
- 17: Strommessvorrichtung
- 18: zentraler Sternpunkt
- 19: Rückleitung
- 21: Rückleitung
- 23: Gleichtaktdrossel
- 25: Kapazitäten
- AE: aktive Elektrode
- NE: Neutralelektrode
- C_{AE}: Auskoppelkondensator
- C_{NE}: Auskoppelkondensator
- C_{SAE}: Kapazität
- C_{SNE}: Kapazität
- R_{NE}: Impedanz Neutralelektrode
- R_{Leck}: Leckstrom-Impedanz

## Patentansprüche

1. HF-Chirurgiegerät (1), umfassend
einen HF-Generator (3), wenigstens ein eine aktive Elektrode (AE) aufweisendes chirurgisches Instrument (5) und eine Neutralelektrode (NE), die mit dem HF-Generator (3) verbindbar sind, wobei das chirurgische Instrument (5) mit dem HF-Generator (3) über eine erste Leitung (7) und die Neutralelektrode (NE) mit dem HF-Generator (3) über eine zweite Leitung (9) verbindbar ist, wobei zumindest die erste Leitung (7) mit einer Abschirmung (11, 13) versehen ist, und wobei an dem distalen Ende der ersten Leitung (7) ein Auskoppelkondensator (C_{AE}, C_{NE}) in Serie zwischen dem HF-Generator (3) und dem chirurgischen Instrument (5) angeordnet ist, wobei die Abschirmung der ersten Leitung (AE, NE) über einen Erdungspfad (15) geerdet ist.

2. HF-Chirurgiegerät nach Anspruch 1, **dadurch gekennzeichnet, dass** eine Strommessvorrichtung (17) in dem Erdungspfad (15) vorgesehen ist, die einen über den Erdungspfad (15) fließenden Leckstrom erfasst.

3. HF-Chirurgiegerät nach einem der vorangehenden Ansprüche, **dadurch gekennzeichnet, dass** die erste Leitung (7) und die zweite Leitung (9) jeweils mit einer Abschirmung (11, 13) versehen sind.

4. HF-Chirurgiegerät nach Anspruch 3, **dadurch gekennzeichnet, dass** an dem distalen Ende der ersten Leitung (7) und an dem distalen Ende der zweiten Leitung (9) ein Auskoppelkondensator (C_{AE}, C_{NE}) vorgesehen ist.

5. HF-Chirurgiegerät nach einem der Ansprüche 1 oder 2, **dadurch gekennzeichnet, dass** ausschließlich an dem distalen Ende der zweiten Leitung (9) ein Auskoppelkondensator (C_{NE}) vorgesehen ist.

6. HF-Chirurgiegerät nach einem der Ansprüche 1 oder 2, **dadurch gekennzeichnet, dass** ausschließlich an dem distalen Ende der ersten Leitung (7) ein Auskoppelkondensator (C_{AE}) vorgesehen ist.

7. HF-Chirurgiegerät nach einem der vorangehenden Ansprüche, **dadurch gekennzeichnet, dass** die Abschirmungen (11, 13) der Leitungen (7, 9) an einem zentralen Sternpunkt (18) über Rückleitungen (19, 21) mit dem HF-Generator (3) verbindbar sind.

8. HF-Chirurgiegerät nach Anspruch 7, **dadurch gekennzeichnet, dass** in einer Rückleitung (19, 21) wenigstens eine Gleichtaktdrossel (23) vorgesehen ist.

## Claims

1. Electrosurgical device (1), comprising an RF generator (3), at least one surgical instrument (5) having an active electrode (AE), and a neutral electrode (NE), which are connectable to the RF generator (3), wherein the surgical instrument (5) is connectable to the RF generator (3) by way of a first line (7) and the neutral electrode (NE) is connectable to the RF generator (3) by way of a second line (9), wherein the at least the first line (7) is provided with a shield (11, 13), wherein the shield of the first line (AE, NE) is grounded by way of a grounding path (15),
**characterized in that** an output coupling capacitor (C_{AE}, C_{NE}) is arranged in series between the RF generator (3) and the surgical instrument (5) at the distal end of the first line (7).

2. Electrosurgical device according to Claim 1, **characterized in that** a current measuring apparatus (17) is provided in the grounding path (15), said current measuring apparatus detecting a leakage current flowing over the grounding path (15).

3. Electrosurgical device according to any one of the preceding claims, **characterized in that** the first line (7) and the second line (9) are each provided with a shield (11, 13).

4. Electrosurgical device according to Claim 3, **characterized in that** an output coupling capacitor (C_{AE}, C_{NE}) is provided at the distal end of the first line (7) and at the distal end of the second line (9).

5. Electrosurgical device according to either of Claims 1 and 2, **characterized in that** an output coupling capacitor (C_{NE}) is only provided at the distal end of the second line (9).

6. Electrosurgical device according to either of Claims 1 and 2, **characterized in that** an output coupling capacitor (C_{AE}) is only provided at the distal end of the first line (7).

7. Electrosurgical device according to any one of the preceding claims, **characterized in that** the shields (11, 13) of the lines (7, 9) are connectable to the RF generator (3) via return lines (19, 21) at a central neutral point (18).

8. Electrosurgical device according to Claim 7, **characterized in that** at least one common-mode choke (23) is provided in a return line (19, 21).

## Revendications

1. Appareil chirurgical HF (1), comprenant un générateur HF (3), au moins un instrument chirurgical (5) qui possède une électrode active (AE), et une électrode neutre (NE) qui peut être reliée au générateur HF (3), l'instrument chirurgical (5) pouvant être relié au générateur HF (3) par le biais d'une première ligne (7) et l'électrode neutre (NE) au générateur HF (3) par le biais d'une deuxième ligne (9), la au moins la première ligne (7) étant munie d'un blindage (11, 13), le blindage de la première ligne (AE, NE) étant relié à la terre par le biais d'un trajet de mise à la terre (15),
**caractérisé en ce qu'**à l'extrémité distale de la première ligne (7), un condensateur de découplage (C_{AE}, C_{NE}) est monté en série entre le générateur HF (3) et l'instrument chirurgical (5).

2. Appareil chirurgical HF selon la revendication 1, **caractérisé en ce qu'**il existe dans le trajet de mise à la terre (15) un dispositif de mesure du courant (17) qui détecte un courant de fuite qui s'écoule par le trajet de mise à la terre (15).

3. Appareil chirurgical HF selon l'une des revendications précédentes, **caractérisé en ce que** la première ligne (7) et la deuxième ligne (9) sont respectivement pourvues d'un blindage (11, 13).

4. Appareil chirurgical HF selon la revendication 3, **caractérisé en ce qu'**un condensateur de découplage (C_{AE}, C_{NE}) se trouve à l'extrémité distale de la première ligne (7) et à l'extrémité distale de la deuxième ligne (9).

5. Appareil chirurgical HF selon l'une des revendications 1 ou 2, **caractérisé en ce qu'**un condensateur de découplage (C_{NE}) se trouve exclusivement à l'extrémité distale de la deuxième ligne (9).

6. Appareil chirurgical HF selon l'une des revendications 1 ou 2, **caractérisé en ce qu'**un condensateur de découplage (C_{AE}) se trouve exclusivement à l'extrémité distale de la première ligne (7).

7. Appareil chirurgical HF selon l'une des revendications précédentes, **caractérisé en ce que** les blindages (11, 13) des lignes (7, 9) peuvent être reliés au générateur HF (3) en un point neutre central (18) par le biais de lignes de retour (19, 21).

8. Appareil chirurgical HF selon la revendication 7, **caractérisé en ce qu'**au moins une bobine de mode commun (23) se trouve dans une ligne de retour (19, 21).
